# EUROPEAN PATENT APPLICATION

(11) **EP 4 183 868 A1**
(43) Date of publication of application: **24.05.2023**
(21) Application number: 21745031.1
(22) Date of filing: 22.01.2021
(51) Int. Cl.: C12N 5/0735, C12N 5/078, C12N 5/0783, A61K 35/17, A61P 35/00

(54) **OFF-THE-SHELF STEM CELLS AND IMMUNE CELLS, AND PHARMACEUTICAL COMPOSITION COMPRISING SAME**

(30) Priority: 23.01.2020 KR 20200009273
(71) Applicant: Kangstem Biotech Co., Ltd., Seoul 06179 (KR)
(72) Inventor: KANG, Kyung-Sun, Seoul 06338 (KR); KWON, Daekee, Seoul 02598 (KR); HAN, Mi-Jung, Seoul 01801 (KR); LEE, Seunghee, Seoul 08797 (KR)
(74) Representative: Betten & Resch
(86) International application number: PCT/KR2021/000942
(87) International publication number: WO 2021/150078

(57) **Abstract**

The present disclosure relates to a differentiation totipotent stem cell having suppressed immune rejection, by having expression of Beta-2 microglobulin (B2M) gene suppressed, and expressing Cluster of Differentiation 24 (CD24), immune cell, and pharmaceutical composition including the same.

## Description

### 1. Field

The present disclosure relates to an off-the-shelf stem cell and immune cell, and a pharmaceutical composition including the same.

### 2. Background

Cancer (malignant tumor) is a major disease with the number one mortality rate in the modern society, and despite numerous studies to date, there is no breakthrough treatment. In the treatment of cancer, treatments using chemotherapeutic agents such as anticancer drugs have been effective to some extent, but such treatments require lots of studies due to the various pathogeneses of cancer and the expression of resistance to anticancer drugs.

The advancement in diagnosis and treatment technologies in recent decades have brought positive results, although limited, such as improved cure rates and functional preservation in cancer treatment, but for many advanced cancers, the 5-year survival rate is hovering at 5 to 50%. These cancers can be characterized by aggressive invasion, lymph node metastasis, distant metastasis, and the occurrence of secondary cancer. In some cancers, despite various studies and treatments, survival rates have not changed significantly over the past 20 years. In recent years, there have been numerous attempts to increase the therapeutic effect through molecular biological approaches to such cancers, and studies on cancer proliferation, metastasis and targeted therapy related to apoptosis have been actively conducted.

Human T cell therapy relies on enriched or modified human T cells to target and kill cancer cells in a patient. In order to increase the ability of T cells to target and kill specific cancer cells, methods have been developed to engineer T cells to express constructs that direct T cells to specific target cancer cells. Chimeric Antigen Receptor (CAR) and engineered T Cell Receptor (TCR) comprising binding domains capable of interacting with specific tumor antigens enable T cells to target and kill cancer cells expressing specific tumor antigens.

Under this background, as a result of intensive research efforts made by the present inventors to develop T cells that exhibit cytotoxicity that can be stably used for cancer treatment without immune rejection, it has been confirmed that cytotoxic T cells having suppressed expression of B2M, CIITA, TCR, PD1 and CTLA4 genes and enhanced expression level of CD24 and CD19CAR genes exhibit effective cancer therapeutic activity. Further, the present inventors completed the present disclosure by confirming that immune cells derived from stem cells having suppressed expression of B2M and CIITA genes, and enhanced expression level of CD24 genes exhibit effective cancer therapeutic activity, and avoid attack from NK cells, thereby resulting in less immune rejection.

### [Prior Art Literature]

### [Patent Literature]

Korean Laid-open Patent no. 10-2019-0130024

### SUMMARY

A purpose of the present disclosure is to provide differentiation totipotent stem cells having suppressed immune rejection.

Another purpose of the present disclosure is to provide immune cells having suppressed immune rejection.

Another purpose of the present disclosure is to provide a pharmaceutical composition that includes such an immune cell.

The present disclosure relates to differentiation totipotent stem cells with suppressed expression of Beta-2 Microglobulin (B2M) genes and that express cluster differentiation 24 (CD24) genes.

The present disclosure relates to immune cells derived from such stem cells.

The present disclosure relates to a pharmaceutical composition for cancer prevention or treatment, including such immune cells.

When using the stem cells and immune cells provided in the present disclosure, immune rejection will not occur, and thus the stem cells and immune cells can not only be used universally but also more economically than existing patient-customized CAR-T therapy, and thus can be used more safely. Therefore, these stem cells and immune cells provided in the present disclosure can be more widely utilized in more effective treatment of illnesses.

### BRIEF DESCRIPTION OF THE DRAWINGS

FIG. 1 illustrates a form of B2M-/- CIITA-/- PDCD1-/- CTLA4-/- iPSC.
FIG. 2 illustrates a B2M-/- CIITA-/- PDCD1-/- CTLA4-/- iPSC genotype. In each drawing, the upper strand is WT allele, and the lower strand is KO allele.
FIG. 3 illustrates a B2M-/- CIITA-/- iPSC genotype (B2M = HLA class I, CIITA = HLA class II).
FIG. 4 is an evaluation result for selecting Novel "Don't eat me gene CD24".
FIG. 5 is a confirmation of production of B2M-/- CIITA-/- PDCD1-/- CTLA4-/- CD24+ CD19 CAR+ iPSC.

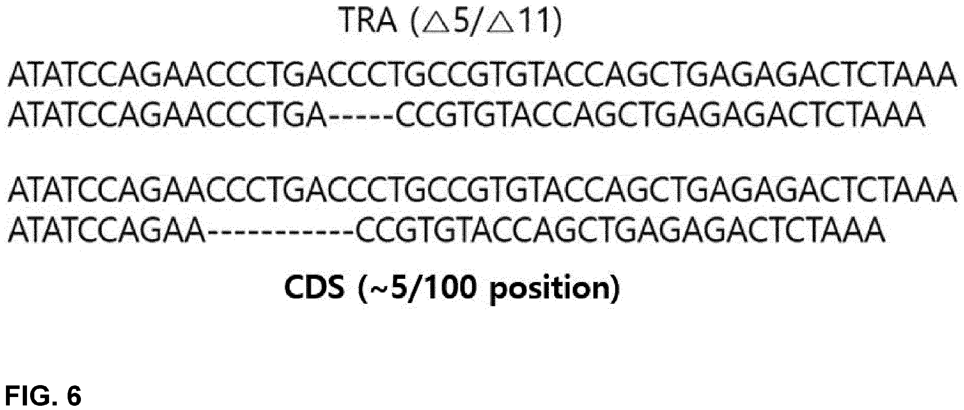

FIG. 6 is sequencing data proving that one or more base deletion occurred in CDS of a TRA gene. That is, it is a confirmation of production of B2M-/- CIITA-/- PDCD1-/- CTLA4-/- TRA-/- CD24+ CD19 CAR+ iPSC.

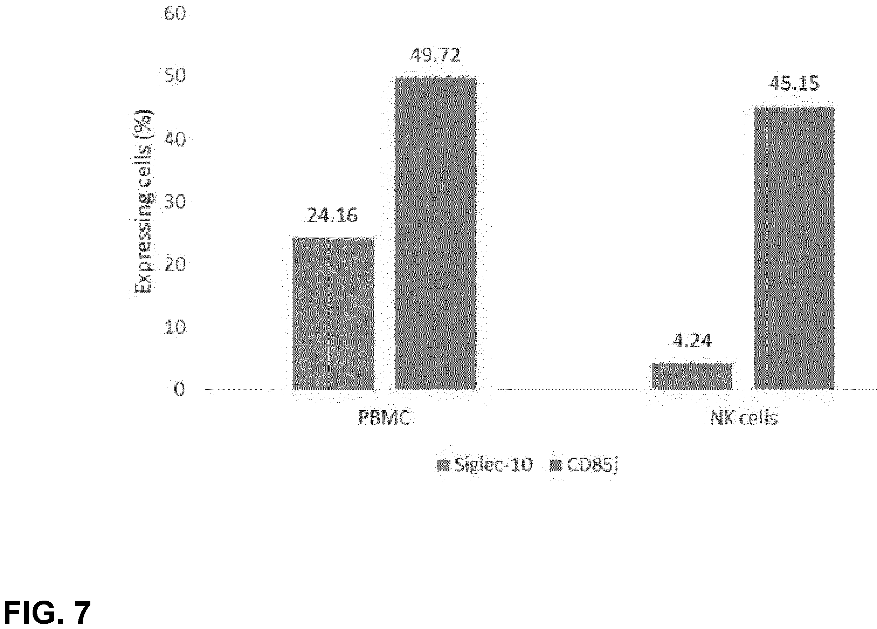

FIG. 7 is a confirmation of inhibitory receptor heterogenicity of PBMC and NK cells.

### DETAILED DESCRIPTION

Hereinbelow, the present disclosure will be described in detail.

The present disclosure relates to differentiation totipotent stem cells with suppressed expression of a Beta-2 Microglobulin gene (B2M) and that express a Cluster of Differentiation 24 (CD24) gene.

The B2M gene is a gene that plays the role of HLA class I. If the expression of the B2M gene is suppressed/deleted, the activity of HLA class I is suppressed/deficient.

The CD24 gene is a new Don't eat me gene. A cell with deficient activity of HLA class I is attacked by a natural killer cell after transplant, but the aforementioned Don't' eat me gene defends against the attack of the natural killer cell.

The differentiation totipotent stem cell of the present disclosure has suppressed expression of the B2M gene and express the CD24 gene, and may thereby not exhibit immune rejection.

Suppression of gene expression means suppression or deletion of expression, which may be by a well-known method in the art, for example, CRISPR and like may be used.

In the case of using CRISPR, in order to suppress the expression of the B2M gene, sgRNA of sequence number 1 may be used, for example.

The CD24 gene may be expressed endogenously or exogenously, and its expression may be increased. In a case where a foreign gene is introduced, it may be, for example, a human-derived gene, and more specifically, a gene consisting of sequence numbers of 6 (NM_013230).

Gene introduction may be by a well-known method in the art, for example, electroporation and the like may be used, but there is no limitation thereto.

When introducing a gene, various vectors may be used, including well known plasmids, viral vectors, non-viral vectors, and the like.

The differentiation totipotent stem cell of the present disclosure may be an embryonic stem cell or induced pluripotent stem cell (iPSC).

The differentiation totipotent stem cell of the present invention may be one with further suppressed expression of at least one gene, selected from a group consisting of Class II, major histocompatibility complex, transactivator (CIITA), Programmed cell death protein 1 (PDCD1), cytotoxic T-lymphocyte-associated protein 4 (CTLA4) and T-cell receptor (TCR).

CIITA is a gene that plays the role of HLA class II, and when the CIITA gene is suppressed/deleted, the activity of HLA class II is inhibited/deficient. As the aforementioned gene is suppressed, the immune rejection is further suppressed.

PDCD1 is one type of T cell inhibitory receptor. When a cancer cell binds to a PD1 of a T cell, the function of the T cell is suppressed. By suppressing the aforementioned gene, it is possible to prevent the activity of the immune cell differentiated from the aforementioned stem cell from being suppressed by the cancer cell.

CTLA4 is one type of T cell inhibitory receptor. When a cancer cell binds to a CTLA4 of a T cell, the function of the T cell is suppressed. By suppressing the aforementioned gene, it is possible to prevent the activity of the immune cell differentiated from the aforementioned stem cell from being suppressed by cancer cell.

TCR is a gene that plays the role of T cell antigen recognition receptor. By suppressing the aforementioned gene, it is possible to prevent the immune cell differentiated from the aforementioned stem cell from causing Graft-versus-host disease (GVHD). TCR may be, for example, a T cell receptor Alpha (TRA).

Suppression of gene expression means suppression or deletion of expression, which may be by a well-known method in the art, for example, CRISPR and like may be used.

In the case of using CRISPR, sgRNA of sequence number 2 may be used in order to suppress expression of CIITA, sgRNA of sequence number 3 may be used in order to suppress expression of PDCD1, sgRNA of sequence number 4 may be used in order to suppress expression of CTLA4, and sgRNA of sequence number 5 may be used in order to suppress expression of TCR.

The differentiation totipotent stem cell of the present disclosure may be one that expresses a cancer cell surface antigen-specific Chimeric Antigen Receptor (CAR). In such a case, the immune cell differentiated therefrom may exhibit anticancer activity.

CAR is specific for cancer cell surface antigens. The cancer cell surface antigen is not limited to a certain type, and may be the antigen of a specific cancer cell surface of a certain cancer type meant for application. For example, it may be CD19.

The generation of the CAR, the type of each domain, arrangement, sequence and the like may be used without limitation as long as the CAR is one that has an antigen binding domain specific for the cancer cell surface antigen meant for application.

As a specific example of the CD19 CAR, the sequence of the antigen binding domain may be composed of FMC63 clone sequence, for example, the entire sequence may be composed of the sequence of sequence number 7.

The differentiation totipotent stem cell of the present disclosure may be one where expression of various combinations of genes exemplified above are suppressed, or one that expresses CD24 and/or cancer cell surface antigen specific CAR.

For example, it may be one where expression of B2M, CIITA, PDCD1 and CTLA4 gene are suppressed, and that expresses CD24 gene and cancer cell antigen specific CAR, and further one where expression of TCR gene is further suppressed. The TCR may be, for example, TRA.

Further, the present disclosure relates to immune cells differentiated from the aforementioned differentiation totipotent stem cell.

The aforementioned immune cell differentiated from the stem cell may be one that expresses the aforementioned gene that the stem cell expresses and where expression of the gene with suppressed expression is suppressed. Therefore, it may not exhibit immune rejection.

Further, in the case of expressing the cancer cell surface antigen specific CAR, it may exhibit anticancer activity.

The immune cell may be, for example, a cytotoxic T cell, Natural kill (NK) cell or macrophage.

Further, the present disclosure relates to a pharmaceutical composition for prevention or treatment of cancer, including the aforementioned immune cell.

The aforementioned immune cell may be one that expresses cancer cell surface antigen specific CAR.

There is no limitation to the type of antigen binding domain that may be used in CAR, and the antigen binding domain suitable to the type of cancer meant for application may be used, and therefore, the pharmaceutical composition of the present disclosure may exhibit anticancer activity to various types of cancers. As an example, it may be solid cancer or blood cancer, and as another example, it may be solid cancer such as pancreatic cancer, breast cancer, prostate cancer, brain tumor, head and neck carcinoma, melanoma, myeloma, liver cancer, gastric cancer, colon cancer, bone cancer, uterine cancer, ovarian cancer, rectal cancer, esophageal cancer, small intestine cancer, anal muscle cancer, colon cancer, fallopian tube carcinoma, endometrial carcinoma, cervical carcinoma, vaginal carcinoma, vulvar carcinoma, Hodgkin's disease, bladder cancer, kidney cancer, ureter cancer, renal cell carcinoma, renal pelvic carcinoma, and central nervous system tumor, and as another example, it may be blood cancer such as leukemia.

The pharmaceutical composition of the present disclosure may further include a pharmaceutically acceptable carrier, excipient, or diluent commonly used in the preparation of pharmaceutical compositions, and the carrier may include a non-naturally occurring carrier. Examples of the carrier, excipient, and diluent include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oils.

Further, the pharmaceutical composition may be formulated in forms of tablets, pills, powders, granules, capsules, suspensions, solutions, emulsions, syrups, sterilized aqueous solutions, non-aqueous solvents, suspensions, emulsions, freeze-dried preparations, transdermal absorbents, gels, lotions, ointments, creams, patches, cataplasmas, pastes, sprays, skin emulsions, skin suspensions, transdermal delivery patches, drug-containing bandages or suppositories according to commonly used methods. Specifically, when formulating, the pharmaceutical composition may be prepared using diluents or excipients such as fillers, weight agents, binders, wetting agents, disintegrants, and surfactants that are commonly used. Solid materials for oral administration include tablets, pills, powders, granules, capsules, but there is no limitation thereto. Such solid materials may be produced by mixing at least one or more excipients, for example, starch, calcium carbonate, sucrose, lactose, gelatin and the like.

Further, besides simple excipients, lubricants such as magnesium stearate and talc may also be used. Besides liquids and liquid paraffins for oral use, various excipients, such as wetting agents, sweetening agents, fragrances, preservatives, and the like may be added. Formulations for parenteral administration include sterile aqueous solutions, non-aqueous solutions, suspensions, emulsions, lyophilized formulations, and suppositories. Propylene glycol, polyethylene glycol, vegetable oils such as olive oil, injectable esters such as ethyloleate and the like may be used as the non-aqueous solvent and suspending agent. Witepsol, macrogol, tween 61, cacao butter, laurin, glycerogelatin and the like may be used as the base for suppositories.

The pharmaceutical composition of the present disclosure is administered in pharmaceutically effective amounts. The aforementioned term "pharmaceutically effective amount" means an amount that is sufficient to treat a disease with a reasonable benefit/risk ratio applicable to medical treatment, and an effective amount level may be determined depending on factors including subject type and severity, age, sex, activity of the drug, sensitivity to the drug, the time of administration, the route of administration and rate of excretion, duration of treatment, and concurrently used drugs, and other factors well known in the medical field. For example, the pharmaceutical composition may be administered in a dosage of 0.01 to 500 mg/kg per day, more specifically, 10 to 100 mg/kg per day, and this administration may be done once or several times a day.

The pharmaceutical composition may be administered as an individual therapeutic agent or in combination with other therapeutic agents, and may be administered sequentially or simultaneously with conventional therapeutic agents. Further, the pharmaceutical composition may be administered in single or multiple times. Considering all the aforementioned factors, it is important to administer an amount by which maximum effects can be obtained with a minimum amount without any side effects, and the amount may be easily determined by those skilled in the art.

Further, the pharmaceutical composition may be administered orally or parenterally (for example, intravenous, subcutaneous, intraperitoneal or topical), and the administration amount may vary depending on the condition and weight of the patient, the severity of the disease, the form of the drug, the route and time of administration, but may be appropriately selected by those skilled in the art.

Hereinbelow, the present disclosure will be described in detail with reference to examples.

### Example

### 1. Producing Wild type iPSC

Reprogramming genes were introduced into skin cells to produce iPSC.

Specifically, in order to produce a wild type iPSC, a retrovirus or plasmid expressing reprogramming genes (Oct-4, Sox-2, c-Myc, Klf-4) was introduced into human dermal fibroblasts (hDF). A retrovirus of MOI 5-20 was introduced into the hDF in the spinfection method. The plasmid was introduced into the hDF in the electroporation method. The hDF into which the reprograming genes were introduced was cultivated for three weeks under a general iPSC cultivation condition, to consequently form undifferentiated iPSC clusters. The undifferentiated iPSC clusters were mechanically picked using a pipette, and multiplied through subculture, and then used in gene editing experiments. In the present disclosure, the iPSC produced using the retrovirus was mainly used to conduct the gene editing experiment. The sequence used is as shown below.

**[Table 1]**

| Sequence number | Target gene | Coding sequence (CDS) |
|---|---|---|
| 8 | Oct-4 | NM_203289 |
| 9 | Sox-2 | NM_003106 |
| 10 | c-Myc | NM_002467 |
| 11 | Klf-4 | NM_001314052 |

### 2. Producing B2M⁻/⁻ CIITA⁻/⁻ PDCD1⁻/⁻ CTLA4⁻/⁻ iPSC

Four types of sgRNA/Cas9 were introduced into a non-viral plasmid of the aforementioned wild type iPSC in the electroporation method.

sgRNA of sequence number 1 was used for a knockout of the B2M, sgRNA of sequence 2 was used for a knockout of CIITA, sgRNA of sequence number 3 was used for a knockout of PDCD1, and sgRNA of sequence 4 was used for a knockout of CTLA4.

Fifteen single cell-derived colonies were cultured separately, to verify whether there is a knockout through sequencing.

**[Table 2]**

| **Genotype** | **Colony number** | **Ratio (%)** |
|---|---|---|
| B2M^{-/-} CIITA^{-/-} PDCD1^{-/-} CTLA4^{-/-} | 1 | 6.7 |
| B2M^{-/-} CIITA^{-/-} | 2 | 13.3 |
| B2M^{-/-} | 5 | 33.3 |
| Wild type | 7 | 46.7 |

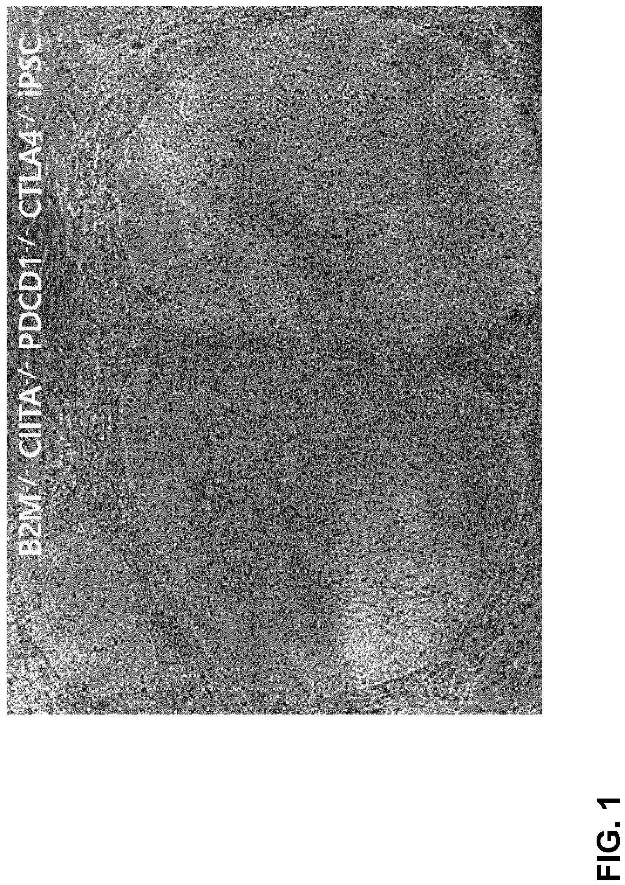

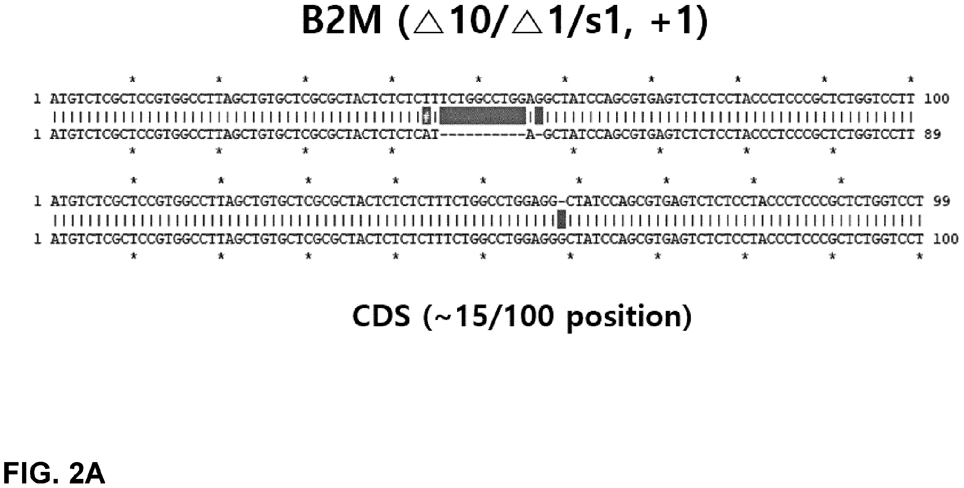

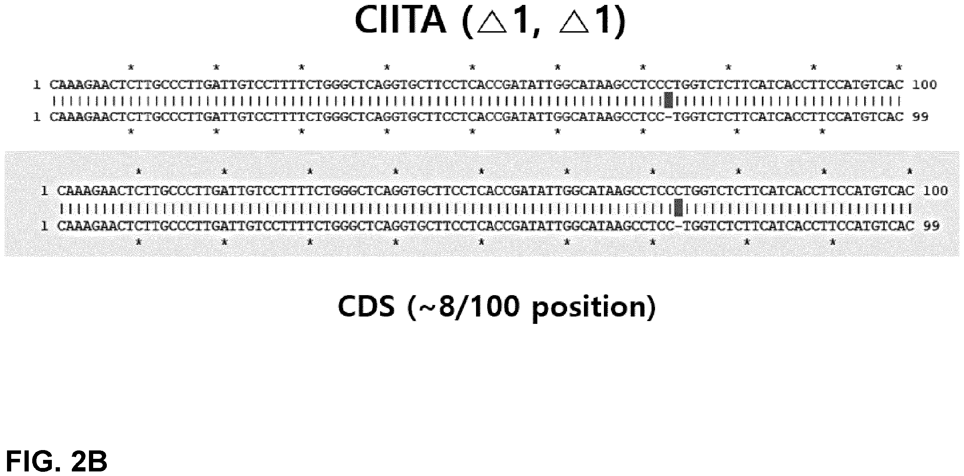

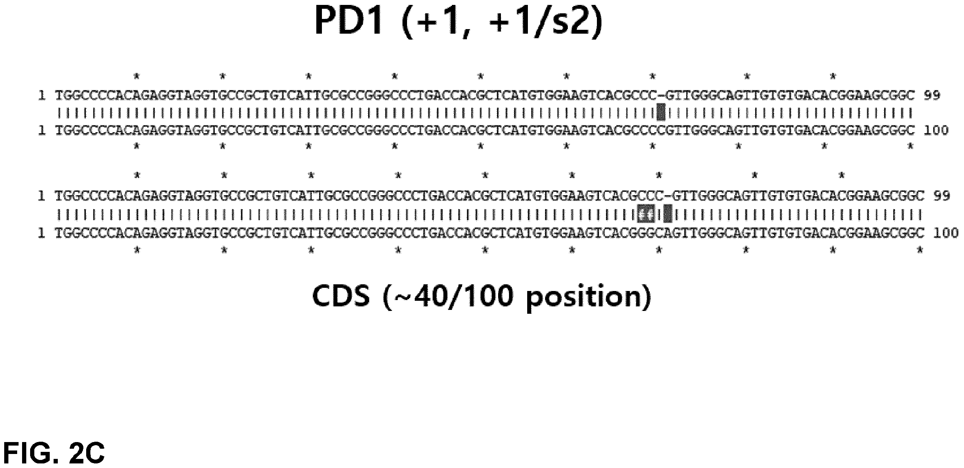

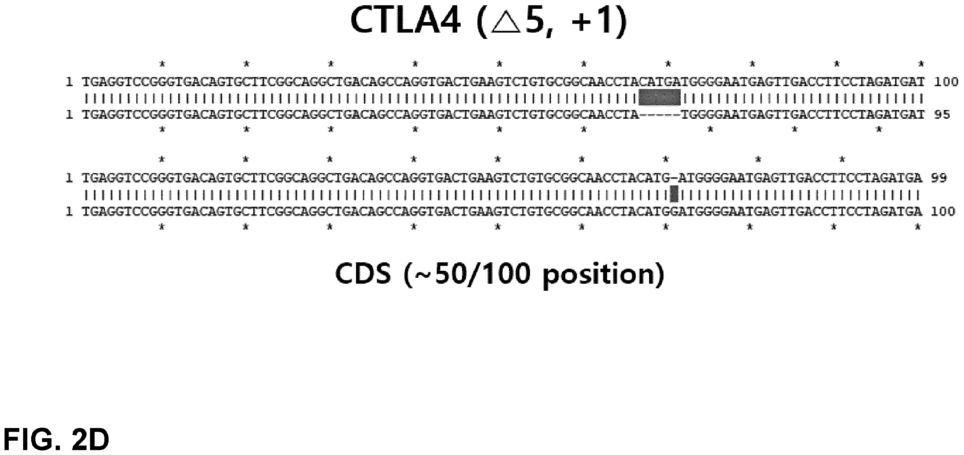

Of the fifteen colonies, it has been confirmed that one is B2M⁻ /⁻ CIITA⁻/⁻ PDCD1⁻/⁻ CTLA4⁻/⁻, which maintained a typical undifferentiated colony form even after gene editing (FIG. 1), and its genotype is as shown in FIG. 2.

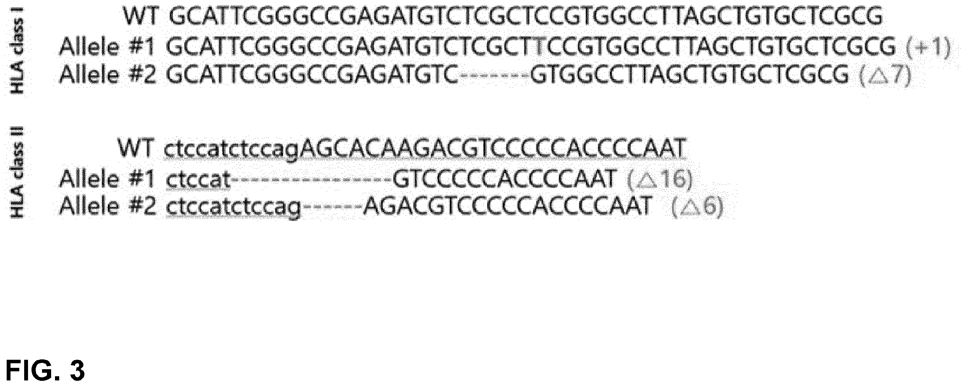

B2M⁻/⁻ CIITA⁻/⁻ iPSC genotype is as shown in FIG. 3.

### 3. Screening Novel Don't eat me gene

In order to screen the Novel Don't eat me gene, CD24, HLA-G (B2M linked), PSG9 (Pregnancy-specific beta-1-glycoprotein 9) gene was cloned in the retrovirus vector (pMX). Each retrovirus was introduced into an HLA class I null (B2M⁻/⁻) iPSC derived differentiated cell by spinfection (MOI=10). By qRT-PCR technique, it was confirmed that each gene was overexpressed 50 times or more compared to the control group. This cell was reacted with a Natural killer cell (NK cell) to compare the degree of cytotoxicity. The sequences used are as shown below.

**[Table 3]**

| Sequence number | Candidate human gene | Coding sequence (CDS) |
|---|---|---|
| 12 | HLA-G | NM_002127 |
| 13 | PSG9 | NM_001301707 |

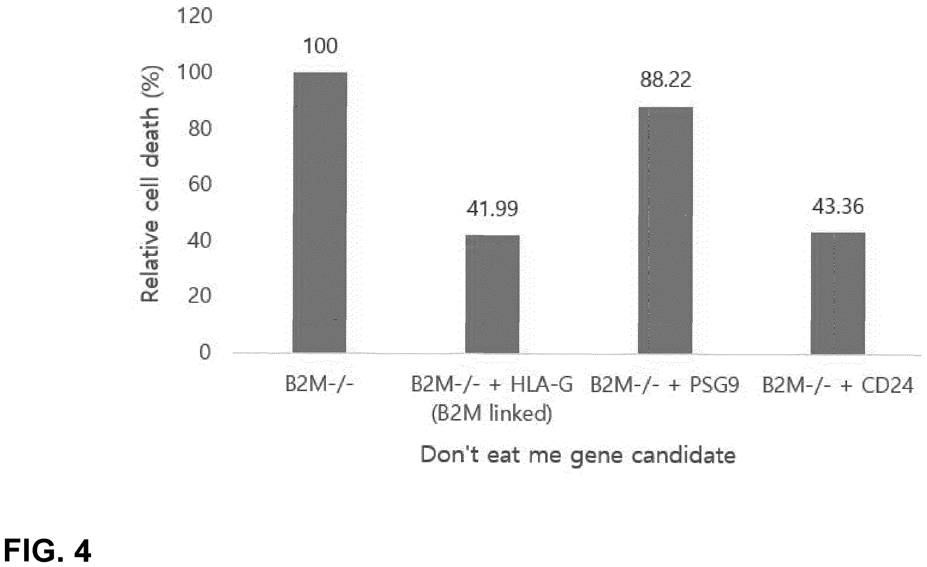

Through this, the CD24 gene was selected, which showed functions of a similar level with HLA-G, an existing well known Don't eat me gene (FIG. 4).

B2M⁻/⁻ cell (HLA class I knockout cell) will be attacked by an NK cell and die (Death rate = approximately 50%). However, when HLA-G, which is a typical Don't eat me gene, is overexpressed in the B2M-/- cell, the NK cell and the HLA-G will interact, and thus will not attack the B2M⁻/⁻ cell. Therefore, the cell death rate decreases by approximately 60% (Death rate = approximately 20%). The present inventors overexpressed CD24 in the B2M⁻/⁻ cell, and confirmed almost the same effect of death rate reduction as HLA-G. This seems to be because an inhibitory receptor that binds with the CD24 exists in the NK cell.

### 4. Producing B2M⁻/⁻ CIITA⁻/⁻ PDCD1⁻/⁻ CTLA4^{-/-} CD24⁺ CD19 CAR⁺ iPSC

Retroviral human CD24 (pMX CD24; sequence number 6, NM_013230) and second generation lentiviral CD19 (FMC63 clone) CAR (pHR CD19 CAR; sequence number 12) were introduced into B2M⁻/⁻ CIITA⁻ /⁻ PDCD1⁻/⁻ CTLA4⁻/⁻ iPSC, and twenty-four single cell derived colonies were individually cultivated, and then whether they were introduced into the cell of CD24, CD19 CAR was verified using PCR.

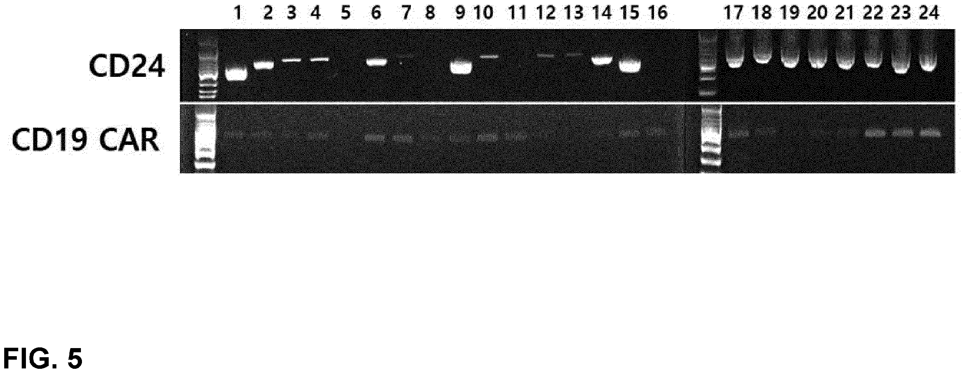

It was confirmed that of the twenty-four colonies, eighteen of them are CD24⁺ CD19 CAR⁺ iPSC (FIG. 5).

### 5. Producing B2M^{-/-} CIITA^{-/-} PDCD1^{-/-} CTLA4^{-/-} TRA^{-/-} CD24⁺ CD19 CAR⁺ iPSC

The T cell receptor (TCR) consists of TRA and TRB gene. By introducing CRISPR/Cas9 for TRA knockout into B2M-/- CIITA⁻/⁻ PDCD1-/- CTLA4⁻/⁻ CD24⁺ CD19 CAR⁺ iPSC by the electroporation method, TRA⁻/⁻ was added. For this purpose, sgRNA of sequence number 5 was used. Consequently, B2M-/- CIITA-/- PDCD1-/- CTLA4-/- TRA-/- CD24⁺ CD19 CAR⁺ iPSC has been produced.

### 6. Confirming heterogenicity of NK cell

In order to analyze the mechanism of the functions of the Novel Don't eat me gene, CD24, the inhibitory receptor expressed in the peripheral blood mononuclear cell (PBMC) and NK cell was analyzed by FACS. Consequently, the PBMC and NK cell expressed CD85j, which is the receptor that binds with HLA-G. Further, apart from this, Siglec-10, that is a receptor that binds with CD24, was expressed. Taken together, it may be described that the CD 24 overexpressed in the HLA class I null cell binds with the Siglec-10 of the NK cell, to function as the don't eat me gene.

## Claims

1. A differentiation totipotent stem cell with suppressed expression of a Beta-2 microglobulin (B2M) and that expresses a cluster of differentiation 24 (CD24) gene.

2. The differentiation totipotent stem cell according to claim 1, wherein the expression of at least one gene selected from a group consisting of class II, major histocompatibility complex, transactivator (CIITA), Programmed cell death protein 1 (PDCD1), cytotoxic T-lymphocyte-associated protein 4 (CTLA4) and T-cell receptor (TCR) is suppressed.

3. The differentiation totipotent stem cell according to claim 1, that expresses a cancer cell surface antigen specific Chimeric Antigen Receptor (CAR).

4. The differentiation totipotent stem cell according to claim 1, wherein the expression of B2M, CIITA, PDCD1 and CTLA4 gene is suppressed, and CD24 gene and cancer cell surface antigen specific CAR is expressed.

5. The differentiation totipotent stem cell according to claim 4, wherein the expression of TCR is suppressed.

6. The differentiation totipotent stem cell according to claim 1, wherein the TCR is T cell receptor Alpha (TRA).

7. The differentiation totipotent stem cell according to claim 1, wherein the cancer cell surface antigen is Cluster of Differentiation 19 (CD19).

8. An immune cell derived from the stem cell according to any one of claims 1 to 7.

9. The immune cell according to claim 8, that is cytotoxic T cell, Natural Kill (NK) cell, or macrophage.

10. A pharmaceutical composition for cancer prevention or treatment, comprising the immune cell of claim 3.

11. The pharmaceutical composition according to claim 10, wherein the cancer is solid cancer or blood cancer.

12. The pharmaceutical composition according to claim 11, wherein the solid cancer is a type of cancer selected from a group consisting of pancreatic cancer, breast cancer, prostate cancer, brain tumor, head and neck carcinoma, melanoma, myeloma, liver cancer, gastric cancer, colon cancer, bone cancer, uterine cancer, ovarian cancer, rectal cancer, esophageal cancer, small intestine cancer, colon cancer, fallopian tube cancer, vaginal cancer, vulvar cancer, Hodgkin's disease , bladder cancer, kidney cancer, ureter cancer, renal cell carcinoma, renal pelvic cancer, central nervous system tumor, and a combination thereof.

13. The pharmaceutical composition according to claim 11, wherein the blood cancer is leukemia.
